# EUROPEAN PATENT SPECIFICATION

(11) **EP 1 979 370 B1**
(45) Date of publication and mention of the grant of the patent: **17.03.2010**
(21) Application number: 07703919.6
(22) Date of filing: 16.01.2007
(51) Int. Cl.: C07K 1/02, C07K 1/06

(54) **SELECTIVE ENZYMATIC HYDROLYSIS OF C-TERMINAL tert-BUTYL ESTERS OF PEPTIDES**
SELEKTIVE ENZYMATISCHE HYDROLYSE VON C-TERMINALEN tert-BUTYL-PEPTIDESTERN
HYDROLYSE ENZYMATIQUE SÉLECTIVE D'ESTERS tert-BUTYLIQUES C-TERMINAUX DE PEPTIDES

(30) Priority: 17.01.2006 EP 06100420
(43) Date of publication of application: 15.10.2008
(73) Proprietor: N.V. Organon, 5349 AB Oss (NL)
(72) Inventor: EGGEN, Ivo, Franci, NL-5340 BH Oss (NL); BOERIU, Carmen, G., NL-6703 EV Wageningen (NL)
(74) Representative: Quelle-Fontijn, Monique
(86) International application number: PCT/EP2007/050409
(87) International publication number: WO 2007/082890

(56) References cited:
- EP-A- 0 310 012
- US-A1- 2004 092 434
- JENS UWE KLEIN ET AL.: "The Applicability of Subtilisin Carlsberg in Peptide Synthesis" JOURNAL OF PEPTIDE SCIENCE, vol. 6, no. 11, 13 November 2000 (2000-11-13), pages 541-549, XP002401403
- MARLEN SCHMIDT ET AL.: "Enzymatic Removal of Carboxyl Protecting Groups. 1. Cleavage of the tert-Butyl Moiety" JOURNAL OF ORGANIC CHEMISTRY, vol. 70, no. 9, 29 April 2005 (2005-04-29), pages 3737-3740, XP002401402 cited in the application
- BARBAS C F ET AL: "A SEARCH FOR PEPTIDE LIGASE: COSOLVENT-MEDIATED CONVERSION OF PROTEASE TO ESTERASES FRO IRREVERSIBLE SYNTHESIS OF PEPTIDES" JOURNAL OF THE AMERICAN CHEMICAL SOCIETY, AMERICAN CHEMICAL SOCIETY, WASHINGTON, DC, US, vol. 110, no. 15, 1988, pages 5162-5166, XP008056136 ISSN: 0002-7863
- BACZKO K ET AL: "New Synthesis of D,L-Fmoc Protected 4-Phosphonomethylphenylalanine Derivatives and their Enzymatic Resolution" TETRAHEDRON, ELSEVIER SCIENCE PUBLISHERS, AMSTERDAM, NL, vol. 52, no. 6, 5 February 1996 (1996-02-05), pages 2021-2030, XP004104434 ISSN: 0040-4020
- TUECHSEN E ET AL: "KINETIC PROPERTIES OF SUBTILISIN TYPE CARLSBERG IN THE CRYSTALLINE STATE" CARLSBERG RESEARCH COMMUNICATIONS, vol. 42, no. 5, 1977, pages 407-420, XP000674318 ISSN: 0105-1938
- MICHAEL SCHULTZ, PETER HERMANN, HORST KUNZ: "Enzymatic Cleavage of tert-Butyl Esters: Thermitase-Catalyzed Deprotection ofPeptides and O-Glycopeptides" SYNLETT, vol. 1992, no. 01, January 1992 (1992-01), pages 37-38,

## Description

The invention relates to a process for selective enzymatic hydrolysis of C-terminal *tert*-butyl esters of peptide substrates in the synthesis of peptides.

In the synthesis of peptides the proper selection of protecting groups is very important. In the chemical synthesis of peptides, elongation of the growing peptide usually occurs in N-terminal direction to avoid racemization of the constituting amino acids. To allow the stepwise elongation of the growing peptide in N-terminal direction, the protecting group for the N-terminal amino function is of a temporary nature. This group is selectively cleaved from the peptide in each cycle of a peptide synthesis without affecting the semi-permanent protecting groups on the functional side chains and the C-terminal. Functional groups on the side chains of the constituting amino acids are usually protected in order to avoid side reactions.
In the chemical synthesis of peptides, either a completely linear approach or a convergent approach may be followed, i.e. an approach in which the final peptide is assembled from protected peptide fragments. The latter approach is generally preferred for longer peptides, since overall yields are intrinsically higher and fragments may be prepared in parallel resulting in a shorter overall synthesis time. In a convergent synthesis, all fragments but the C-terminal fragment must be protected at their C-terminal with a protecting function that can be selectively cleaved. In case of peptide synthesis on a solid support, this function is usually provided by a handle on the support itself. For peptide synthesis on a manufacturing scale, solution-phase synthesis is, however, preferred over solid-phase synthesis. Particularly preferred is a synthesis according to DioRaSSP®, a solution-phase method which combines the advantages of the solid-phase and the classical solution-phase process (EP-A-1,291,356; US 6,864,357; Eggen I. et al., Org. Process Res. Dev. 2005, 9, 98-101; Eggen I. et al., J. Pept. Sci. 2005, 11, 633-641).

The C-terminal ester in a convergent synthesis should be stable under the conditions of the assembly of the peptide and most notably under the conditions of the deprotection of the temporary N-terminal protecting group, which is repeated in each cycle of a peptide synthesis. On the other hand, the N-terminal protecting group and protecting groups of the side chains need to remain unaffected when the ester at the C-terminal is removed prior to the actual fragment coupling. Although a range of esters is known which may be used for protection of the C-terminal of the peptide fragments and several options are available for chemical deprotection, none of the available methods is generally applicable. Most of these esters are of a primary nature, e.g. methyl esters, and thus give rise to diketopiperazine formation at the stage of the deprotected dipeptide. Moreover, introduction of the ester function often requires a relatively strong activation of the carboxylic function resulting in loss of the enantiomeric integrity of the esterified amino acid. Finally, the ester function is often deblocked under acidic or basic conditions that lead to modifications in the actual peptide. On the basis of its high stability, its ease of introduction and its wide commercial availability, *tert*-butyl ester is preferred if selective deprotection in the presence of side-chain protecting groups can be achieved. However, chemical deprotection is generally not applicable for this purpose.

Enzymatic methods have gained interest in peptide synthesis during the past couple of years. Enzymes often show high chemo-, regio- and stereoselectivity. Furthermore, enzymes usually operate under very mild conditions, at neutral pH values and at temperatures of 20-50°C. Thus, under such conditions, acid or base-catalysed side reactions can be circumvented.
It is known in the art that some protecting groups are cleavable by enzymes, which can be used in the deblocking of peptides. In particular esters may be applied, which can be hydrolysed by lipases, esterases, and proteases. Lipases and esterases are usually considered as more generally applicable to protecting group chemistry in peptide synthesis, since a drawback of proteases is that they may also hydrolyse peptide bonds (endopeptidase activity). Recently, it was found that certain lipases and esterases bearing the amino acid motif GGG(A)X (wherein G denotes glycine and X any amino acid; in a few enzymes one glycine is replaced by alanine, A) show mild and selective removal of *tert*-butyl ester protecting groups in amino acid derivatives and related compounds. It was concluded that in particular two enzymes, BsubpNBE (recombinant *p-*nitrobenzyl esterase from *Bacillus subtilis* produced in *E.coli*) and CAL-A (lipase A from *Candida antarctica* (Novozymes)) can be used in synthetic organic chemistry to cleave *tert*-butyl esters from various substrates (Schmidt M. et al., J. Org. Chem. 2005, 70, 3737-3740). The activities of those enzymes have, however, not been tested on C-terminal *tert*-butyl esters of peptides, with the exception of one dipeptide which did not show any activity. The solubility of protected peptides will probably pose a problem in the solvent systems which have been applied for testing the enzyme activities, using in particular toluene, *n*-hexane and diethyl ether as cosolvents. In general, however, protected peptides and especially long protected peptides require polar organic solvents to dissolve (e.g., DMF, NMP, dichloromethane, methanol or acetonitrile).

In spite of the fact that proteases are less preferred the use of a protease named thermitase reportedly has resulted in the effective hydrolysis of peptide esters (Schultz M. et al., Synlett. 1992, 1, 37-38). However, thermitase has not found wide use in peptide synthesis from the time when this was reported. This might, amongst other reasons, be due to its limited commercial availability (family code EC 3.4.21.66), which severely hampers upscaling to industrial scale.
Thus, there is still a need for generally applicable enzymatic processes for removal of C-terminal esters, in particular *tert*-butyl esters, in peptide chemistry, especially for industrial-scale processes.

Moreover, as an alternative to chemical synthesis of peptides, there is a growing interest in the enzymatic synthesis of peptides, in which both coupling and deprotection steps may be mediated by specific enzymes. Since coupling steps mediated by enzymes evoke no racemization as opposed to the chemical alternative, elongation of the growing peptide in enzymatic synthesis of peptides may occur in C-terminal direction. Such a synthesis is in effect a convergent synthesis in which the C-terminal fragment is replaced by an amino acid derivative; it would therefore likewise profit from the availability of generally applicable enzymatic processes for removal of C-terminal esters, in particular *tert*-butyl esters.

Finally, the availability of generally applicable processes for removal of C-terminal esters, in particular *tert*-butyl esters, would be very profitable in peptide syntheses comprising peptide cyclizations which involve the C-terminal of the linear precursor (i.e., head-to-tail and tail-to-side chain cyclizations). Such cyclizations may take place in solution, but also on a solid support when the linear precursor is anchored *via* its N-terminal or a side chain.

A new process has now been found for the selective enzymatic hydrolysis of C-terminal *tert-*butyl esters of peptide substrates in the synthesis of peptides, comprising hydrolysing one or more peptide substrates comprising C-terminal *tert*-butyl esters using the protease subtilisin in any suitable form.
Surprisingly, it has been found that high, up to quantitative, yields of the hydrolysed product can be obtained using the protease subtilisin, while endopeptidase activity is substantially suppressed.

The new process of this invention may conveniently be used in the production of protected or unprotected peptides. The process is in particular favourable in convergent syntheses of peptides.

Preferably, the C-terminal *tert*-butyl ester of the peptide substrates comprises a C-terminal acyl residue which is an α-amino acyl residue from natural or synthetic origin. The C-terminal α-amino acyl residue may be protected or unprotected at the side chain. In particular preferred are C-terminal α-amino acyl residues selected from Ala, protected Cys, protected Asp, protected Glu, Phe, Gly, His, (protected) Lys, Leu, Met, Asn, Gln, (protected) Arg, (protected) Ser, Thr, Val, (protected) Trp and (protected) Tyr, wherein the brackets around the word "protected" mean that the residue can be present in both side-chain protected and unprotected form. The three-letter code for amino acids is used here according to IUPAC nomenclature (IUPAC-IUB Commission (1985) J. Biol. Chem. 260, 14-42).

Preferably, protected or unprotected peptide substrates used in the process of this invention are prepared by solution-phase synthesis. In a further preferred embodiment, protected or unprotected peptide substrates used in the process of this invention are prepared according to DioRaSSP®. Thus, a peptide substrate comprising a C-terminal *tert*-butyl ester is preferably prepared according to this process for rapid solution synthesis of a peptide in an organic solvent or a mixture of organic solvents, the process comprising repetitive cycles of steps (a)-(d):
(a) a coupling step, using an excess of an activated carboxylic component to acylate an amino component,
(b) a quenching step in which a scavenger is used to remove residual activated carboxylic functions, wherein the scavenger may also be used for deprotection of the growing peptide,
(c) one or more aqueous extractions and
optionally, (d) a separate deprotection step, followed by one or more aqueous extractions,
whereby in at least one cycle in process step b an amine comprising a free anion or a latent anion is used as a scavenger of residual activated carboxylic functions. The amine is preferably benzyl β-alaninate or a salt thereof.

The hydrolysis of the process of the present invention may be performed in an aqueous buffer. However, for solubility purposes and/or for suppressing endopeptidase activity, the hydrolysis of the process of the invention is preferably performed in a mixture of an aqueous buffer and one or more organic solvents. Suitable buffers may be selected from buffers which are generally used for transformations using proteolytic enzymes. In particular, the aqueous buffer is a phosphate, borate or TRIS buffer. Polar organic solvents are preferred, and in particular the organic solvent is selected from *N,N*-dimethylformamide (DMF), *N*-methyl-2-pyrrolidone (NMP), dioxane, *N,N-*dimethylacetamide (DMA), dichloromethane (DCM), tetrahydrofuran (THF), acetonitrile, and *tert*-butanol. Particularly preferred is DMF. Accordingly, the percentage of the organic solvent in the mixture may range from 0 to 60% (v/v), preferably from 30 to 60% (v/v). It is especially useful when the percentage of the organic solvent in the mixture is about 50-60% (v/v).
The pH at which the reaction is performed may be selected from the range of 6.5-10, in particular 6.5-8, and preferably the pH is 7.
Reaction temperatures for the hydrolysis may suitably be selected in the range of 20-60°C. Preferred is a reaction temperature of 40°C.
The amount of enzyme may suitably be selected in the range of 1 to 50 wt.% of enzyme related to the peptide substrate.
In a further embodiment of the invention, the hydrolysis is performed by stepwise adding portions of the protease subtilisin (in any suitable form) to the reaction mixture comprising one or more peptide substrates comprising C-terminal *tert*-butyl esters.
The peptide substrates of which the C-terminal *tert*-butyl esters are hydrolysed in the process of the invention, may carry protecting groups in other parts of their peptide sequence.
The hydrolysis can be carried out with one peptide substrate as well as a mixture thereof. It has been established that the performance of the hydrolysis of a mixture of peptide substrates comprising C-terminal *tert*-butyl esters is not significantly reduced by the presence of more than one peptide substrate.

A suitable process according to the present invention is as follows.
To a stirred solution of a *tert*-butyl ester of a peptide in a mixture of a suitable organic solvent (or mixture of organic solvents) and a buffer, subtilisin is added [for instance: to 0.1 mmol of a *tert*-butyl ester of a peptide in a mixture of 2.5 ml DMF and 2.5 ml sodium phosphate buffer (0.1 M, pH 7.0, 40 °C), 5 mg of subtilisin is added]. When substrate conversion has reached a desired level, e.g. higher than 95 % (as determined by HPLC), an amount of ethyl acetate is added. After separation of the aqueous phase, the organic phase is extracted twice with sodium chloride solution and concentrated *in vacuo*. The C-terminally deprotected peptide or peptide fragment is isolated by precipitation with a non-solvent like diethyl ether, methyl *tert*-butyl ether or heptane. In case of an immobilized enzyme, the enzyme is removed by filtration prior to the described work-up procedure.

The protease subtilisin (EC 3.4.21.62) may be used in the process of the invention in any form, thus it may be used in soluble and/or crystallized form, but also in immobilized form or other insoluble form, e.g. in the form of cross-linked enzyme aggregates (CLEA) or cross-linked enzyme crystals (CLEC).

The term 'substrate' herein means an entity which is converted to a product by the protease subtilisin in any form. This product may be a final peptide product whereby if present only the protected side groups have still to be deprotected. Alternatively, this product may also be a peptide fragment which subsequently is reacted with other peptide fragments in a convergent synthesis to obtain a longer peptide with the required final number of amino acids.
A person skilled in the art can easily identify suitable substrates, for instance by performing a simple test hydrolysis of a selected peptide comprising a C-terminal *tert*-butyl ester functionality under suitable conditions as described herein before and following conversion e.g. by HPLC techniques. It has been found by hydrolysis of model dipeptides of the general structure Z-Val-X-OBu*^{t}* in the presence of Alcalase CLEA and 50% v/v of DMF (see for details Example 3) that the reactivity for these model substrates comprising a C-terminal *tert*-butyl ester functionality can be divided into four categories:

| **Conversion** | **X** |
|---|---|
| Good means 50 to 100% conversion to the hydrolysed product | Gln, Ala, Met, Asn, His, Leu, Ser, Arg, Phe, Tyr, Lys(Boc), Thr |
| Mediocre means 5 to 50% conversion to the hydrolysed product | Arg(Pbf), Glu(OBu*^{t}*), Val, Gly, Trp, Trp(Boc) |
| Bad means 1 to 5% conversion | Cystine, Tyr(Bu*^{t}*), Ser(Bu*^{t}*), Asp(OBu*^{t}*) |
| Unresponsive means less than 1% conversion | Ile, Pro, Thr(Bu*^{t}*), His(Trt) |

If the reactivity is indicated to be "unresponsive", also after optimization, the selected peptide is not a substrate according to the invention.
Based on the structure of these model peptide substrates the results are considered indicative of the reactivity of the selected amino acid derivative X towards protease subtilisin in any suitable form. This is corroborated by Example 5 in which it has been tested which amino acid combinations are susceptible to endopeptidase activity of the protease subtilisin. More particularly, model tetrapeptide substrates of the general structure Z-Val-Val-X-Leu-OBu*^{t}* were hydrolysed in the presence of Alcalase CLEA and 50% v/v of DMF (see for details Example 5). Moreover, Example 5 demonstrates that esterase activity in relation to amino acid derivative X is indicative for endopeptidase activity in relation to that amino acid derivative X. However, it is emphasized that under optimized conditions of the process of the present invention endopeptidase activity is significantly lower than esterase activity.
It is established that the process is very suitable to prepare short peptides, such as dipeptides, tripeptides, and tetrapeptides. Furthermore, based on the above-mentioned arguments on the basis of Examples 3 and 5 a skilled man is also able to prepare longer peptides comprising amino acid derivatives which are expected to have bad or mediocre reactivity in any but the C-terminal position of the peptide. In this sense, D-amino acid derivatives can be considered as unresponsive amino acid derivatives and thus peptides may be prepared as well with D-amino acids in any but the C-terminal position.

The term 'protected' means that the functional groups (within the peptide) are protected with suitable protecting groups. A person skilled in the art will know which type of protection to select for which type of functional group. For example, amine functions present in the compounds may be protected during the synthetic procedure by an N-protecting group, which means a group commonly used in peptide chemistry for the protection of an a-amino group, like the ter-t-butyloxycarbonyl (Boc) group, the benzyloxycarbonyl (Z) group, or the 9-fluorenylmethyloxycarbonyl (Fmoc) group. Overviews of amino protecting groups and methods for their removal is given in Geiger R. and König W. (1981) in Peptides: Analysis, Synthesis, Biology, Vol 3, Gross E. and Meienhofer, J., eds, Academic Press, New York, pp. 1-99, and Peptides: Chemistry and Biology, Sewald N. and Jakubke H.-D., eds, Wiley-VCH, Weinheim, 2002, pp. 143 - 154. Functions of the *tert*-butyl type or functions of similar lability are preferred for the protection of other functional groups on the side chains; these include - but are not limited to - *tert*-butyl (Bu^{t}) for the protection of the Asp, Glu, Ser, Thr and Tyr side chains, *tert-*butoxycarbonyl (Boc) for the protection of the Lys and Trp side chains, trityl (Trt) for the protection of the Asn, Gln and His side chains and 2,2,5,7,8-pentamethylchromane-6-sulfonyl (Pmc) or 2,2,4,6,7-pentamethyldihydrobenzofurane-5-sulfonyl (Pbf) for the protection of the Arg side chain [Barany, G. and Merrifield, R.B. (1980) in: 'The Peptides', vol. 2 (Gross, E. and Meienhofer, J., eds.) Academic Press, New York, pp. 1-284; for Trp(Boc): Franzén, H. et al. (1984) J. Chem. Soc., Chem. Commun., 1699-1700; for Asn(Trt) and Gln(Trt): Sieber, P. and Riniker, B. (1991) Tetrahedron Lett. 32, 739-742; for His(Trt): Sieber, P. and Riniker, B. (1987) Tetrahedron Lett. 28, 6031-6034; for Pmc: Ramage, R. and Green, J. (1987) Tetrahedron Lett. 28, 2287-2290; for Pbf: Carpino, L.A. et al. (1993) Tetrahedron Lett. 34, 7829-7832].

The invention is further illustrated by the following examples.

### EXAMPLES

The tetrapeptide of Example 2 and the tetrapeptide of Example 5 wherein X=Met have been prepared according to DioRaSSP® (EP-A-1,291,356; US 6,864,357; Org. Process Res. Dev. 2005, 9, 98-101; Eggen I. et al., J. Pept. Sci. 2005, 11, 633-641), whereas the dipeptides, tripeptides, and the tetrapeptide of Example 5 wherein X=Arg have been produced according to conventional solution phase methods for peptide synthesis. The other tetrapeptides of Example 5 are prepared according to conventional solution phase methods applying a split and mix protocol. Split and mix protocol has been applied in order to reduce the amount of preparative work.

The free enzyme subtilisin A (S. Carlsberg) used in Example 1 was purchased from Sigma Aldrich.
The free enzyme subtilisin A used in the other Examples was purchased from Novozymes. Alcalase CLEA was obtained from CLEA Technologies B.V., Delft, The Netherlands.

### Example 1

### Free subtilisin

### General procedure Subtilisin A:

0.1 mmol of each of the peptides was dissolved in a thermostated mixture of 2.5 ml DMF and 2.5 ml phosphate buffer 0.1 M pH 7. 5 mg of enzyme were added to the peptide solution. The reaction mixture was incubated at 40°C for a total of two hours. The reaction mixtures were analysed after 2 hours using HPLC.
(Some selected results of good subtilisin substrates are shown.)

**Table 1**

| **Substrate** | **Product / Substrate** **(% area)** | **Product** |
|---|---|---|
| Z-Val-Ala-OBu*^{t}* | 100/0 | Z-Val-Ala-OH |
| Z-Val-Leu-OBu*^{t}* | 99.1/0.9 | Z-Val-Leu-OH |
| Z-Val-Phe-OBu*^{t}* | 99.4/0.6 | Z-Val-Phe-OH |
| Z-Val-Lys(Boc)-OBu*^{t}* | 96.8/3.2 | Z-Val-Lys(Boc)-OH |
| Z-Ala-Phe-OBu*^{t}* | 94.7/5.3 | Z-Ala-Phe-OH |

### Example 2

0.05 mmol of Boc-Gly-Phe-Phe-Leu-OBu*^{t}* was dissolved in a thermostated mixture of 2.5 ml NMP and 2.5 ml phosphate buffer 0.1M pH 8. To this solution 5 mg of Alcalase CLEA were added. The reaction mixture was incubated at 40°C for a total of 17 hours. Substrate conversion is determined by HPLC. The reaction resulted after 17 h in >95 % yield of Boc-Gly-Phe-Phe-Leu-OH.

### Example 3

### Deprotection of model dipeptides by Alcalase CLEA and subtilisin A

### General procedure Subtilisin A:

0.1 mmol of each of the peptides was dissolved in 2.5 ml DMF. 5 mg of enzyme were dissolved in 2.5 ml phosphate buffer 0.1 M pH 7 and were added to the peptide solution. The reaction mixture was incubated at 40°C for a total of six hours. The reaction mixtures were analysed after 6 hours using HPLC and LC/MS.

### General procedure Alcalase CLEA:

0.1 mmol of each of the peptides was dissolved in 2.5 ml DMF. To this solution 5 mg of enzyme were added along with 2.5 ml phosphate buffer 0.1M pH 7. The reaction mixture was incubated at 40°C for a total of six hours. The reaction mixtures were analysed after 6 hours using HPLC and LC/MS.

**Table 2**

| **Substrate** | **Product *vs*. Substrate (% area)** | |
|---|---|---|
| | **Alcalase CLEA** | **Subtilisin A** |
| **50-100% Conversion using Alcalase CLEA in 50 v/v% DMF** **(Good conversion)** | | |
| Z-Val-Gln-OBu*^{t}* | 100/0 | 100/0 |
| Z-Val-Ala-OBu*^{t}* | 100/0 | 100/0 |
| Z-Val-Met-OBu*^{t}* | 99.8/0.2 | 100/0 |
| Z-Val-Asn-OBu*^{t}* | 100/0 | 99.6/0.4 |
| Z-Val-His-OBu*^{t}* | 99.7/0.3 | 73.5/26.5 |
| Z-Val-Leu-OBu*^{t}* | 98/2 | 100/0 |
| Z-Val-Ser-OBu*^{t}* | 98.3/1.7 | 100/0 |
| Z-Val-Arg-OBu*^{t}* | 95.9/4.1 | 95.5/4.5 |
| Z-Val-Phe-OBu*^{t}* | 95.2/4.8 | 95.5/4.5 |
| Z-Val-Tyr-OBu*^{t}* | 91.4/8.6 | 99.9/0.1 |
| Z-Val-Lys(Boc)-OBu*^{t}* | 85.9/14.1 | 94.8/5.2 |
| Z-Val-Thr-OBu*^{t}* | 56.7/43.3 | 74.9/25.1 |

| **5-50% Conversion using Alcalase CLEA in 50 v/v% DMF** **(Mediocre conversion)** | | |
|---|---|---|
| Z-Val-Arg(Pbf)-OBu*^{t}* | 45.5/54.5 | 82.7/17.3 |
| Z-Val-Glu(OBu*^{t}*)-OBu*^{t}* | 39.5/60.5 | 54.5/45.5 |
| Z-Val-Val-OBu*^{t}* | 29.2/70.8 | 15.4/84.6 |
| Z-Val-Gly-OBu*^{t}* | 16.4/83.6 | 12.2/87.8 |
| Z-Val-Trp-OBu*^{t}* | 10.6/89.4 | 6.5/93.5 |
| Z-Val-Trp(Boc)-OBu*^{t}* | 9.4/90.6 | 27.9/72.1 |

| **1-5% Conversion using Alcalase CLEA in 50 v/v% DMF** **(Bad conversion)** | | |
|---|---|---|
| (Z-Val-Cys-OBu*^{t}*)₂ | 2.5/97.5 | 12.7/87.3 |
| Z-Val-Tyr(Bu*^{t}*)-OBu*^{t}* | 2.5/97.5 | 6.3/93.7 |
| Z-Val-Ser(Bu*^{t}*)-OBu*^{t}* | 1.5/98.5 | 1.7/98.3 |
| Z-Val-Asp(OBu*^{t}*)-OBu*^{t}* | 0.9/99.1 | 2.4/97.6 |

| **Less than 1% conversion in 50 v/v% DMF** **(unresponsive)** | | |
|---|---|---|
| Z-Val-Ile-OBu*^{t}* | traces of product | 0/100 |
| Z-Val-Pro-OBu*^{t}* | 0/100 | 0/100 |
| Z-Val-Thr(Bu*^{t}*)-OBu*^{t}* | 0/100 | 0/100 |
| Z-Val-His(Trt)-OBu*^{t}* | 0/100 | 0/100 |

### Example 4

### Optimization of the amount of enzyme for a good and a mediocre peptide substrate

### General procedure

0.1 mmol of each of the peptides was dissolved in 2.5 ml DMF. Subtilisin A was dissolved in 2.5 ml phosphate buffer 0.1 M pH 7 and added to the peptide solution. The reaction mixture was incubated at 40°C. The reaction mixtures were sampled at the indicated reaction times and analysed by HPLC. When needed extra portions of enzyme were added at 2-hour intervals.

**Table 3**

| **Substrate** | **Amount of enzyme** | **Reaction time** | **Product *vs*. Substrate (% area)** |
|---|---|---|---|
| Z-Val-Gly-OBu*^{t}* | 5 mg | 2 h | 9.9/90.1 |
| | | 4 h | 11.5/88.5 |
| | | 6 h | 11.9/88.1 |
| | 10 mg | 2 h | 24.1/75.9 |
| | | 4 h | 26.7/73.3 |
| | | 6 h | 27.3/72.7 |
| | 2x5 mg | 4 h | 20.3/79.7 |
| | 3x5 mg | 6 h | 56.5/43.5 |
| | | | |
| Z-Val-Leu-OBu*^{t}* | 5 mg | 2 h | 100/0 |
| | 2.5 mg | 2 h | 100/0 |
| | 0.5 mg | 2 h | 56.9/43.1 |
| | | 4 h | 66.1/33.9 |
| | | 6 h | 67.9/32.1 |
| | 2x0.5 mg | 4 h | 98.5/1.5 |
| | 3x0.5 mg | 6 h | 99.6/0.4 |

### Example 5

### Identification of peptide substrates susceptible to endopeptidic cleavage.

### General procedure Alcalase CLEA:

0.1 mmol of each of the tetrapeptides (or tetrapeptide mixtures) were dissolved in 2.5 ml DMF. To this solution 5 mg of enzyme were added along with 2.5 ml phosphate buffer 0.1 M pH 7 and were added to the previous solution. The reaction mixture was incubated at 40°C. Reaction mixtures were analysed after 2 hours and 6 hours by HPLC. In case the reaction had not reached completion additional portions of enzyme [5 mg each] were added at the end of six hours and overnight (approx. 24 hours of reaction). The last aliquot was analysed by LC/MS to verify the presence of the desired compound and of any impurities being Z-Val-Val-X-OH and/or H-X-Leu-OH.

**Table 4**

| **X in Z-Val-Val-X-Leu-OBu***^{t}* | **Reaction time** | **Ratio endopeptidase Impurity *vs*. Desired Product (% area)** | **Ratio Desired Product *vs*. Substrate (% area)** |
|---|---|---|---|
| Met | 2 h | 2.6/97.4 (Z-Val-Val-X-OH) | 28.8/71.2 |
| | 6 h | 2.8/97.2 (Z-Val-Val-X-OH) | 63:4/36.6 |
| | 24 h | 72/28 (Z-Val-Val-X-OH) | 100/0 |
| | | 5.9/94.1 (H-X-Leu-OH) | |
| Arg | 2 h | n.d. | 97.7/2.3 |
| | 6 h | n.d. | 100/0 ¹⁾ |
| Gln (mix A) | 2 h | 3.5/96.5 (Z-Val-Val-X-OH) | 82.5/17.5 |
| | 6 h | 8.6/91.4 (Z-Val-Val-X-OH) | 100/0 ¹⁾ |
| Leu (mix A) | 2h | 2.9%97.1 (Z-Val-Val-X-OH) | 94.4/5.6 |
| | 6 h | 10.2/89.8 (Z-Val-Val-X-OH) | 100/0 ¹⁾ |
| Ala (mix A) | 2 h | 0.8/99.2 (Z-Val-Val-X-OH) | 89.8/10.2 |
| | 6 h | 2.9/97.1 (Z-Val-Val-X-OH) | 100/0 ¹⁾ |
| Ser (mix A) | 2 h | n.d. | 98.3/1.7 |
| | 6 h | n.d. | 100/0 ¹⁾ |
| Asn (mix B) | 2 h | n.d. | 100/0 |
| | 6 h | 0.7/99.3 (H-X-Leu-OH) | 100/0 |
| | 24 h 24 h | 3.1/96.9 (H-X-Leu-OH) | 100/0 |
| | | 6.8/93.2 (Z-Val-Val-X-OH) | |
| | 48 h | 39.1/60.9 (Z-Val-Val-X-OH) | 100/0 |
| | | 11.3/88.7 (H-X-Leu-OH) | |
| Tyr (mix B) | 2 h | n.d. | 42.1/57.9 |
| | 6 h | n.d. | 90.1/9.9 |
| | 24 h | n.d. | 98.2/1.8 |
| | 48 h | n.d. | 100/0 |
| Phe (mix B) | 2h | n.d. | 23/77 |
| | 6 h | n.d. | 81/19 |
| | 24 h | 13.6/86.4 (Z-Val-Val-X-OH) | 97.9/2.1 |
| | 48 h | 50.9/49.1 (Z-Val-Val-X-OH) | 100/0 |
| Lys(Boc) (mix B) | 2 h | 1.1/98.9 (Z-Val-Val-X-OH) | 61.1/38.9 |
| | 6 h | 4.4/95.6 (Z-Val-Val-X-OH) | 90.8/9.2 |
| | 24 h | 18.5/81.5 (Z-Val-Val-X-OH) | 95.7/4.3 |
| | 48 h | 63.3/36.7 (Z-Val-Val-X-OH) | 100/0 |
| Arg(Pbf) (mix C) | 2 h | 0.5/99.5 (Z-Val-Val-X-OH) | 31.5/68.5 |
| | 6 h | 0.7/99.3 (Z-Val-Val-X-OH) | 49.2/50.8 |
| | | 0.1/99.9 (H-X-Leu-OH) | |
| | 24 h | 3.2/96.8 (Z-Val-Val-X-OH) | 84.5/15.6 |
| | | 1/99 (H-X-Leu-OH) | |
| | 48 h | 20.1/79.9 (Z-Val-Val-X-OH) | 100/0 |
| | | 2.3/97.7 (H-X-Leu-OH) | |
| Thr (mix C) | 2 h | n.d. | cannot be determined ²⁾ |
| | 6 h | 0.9/99.1 (Z-Val-Val-X-OH) | cannot be determined ²⁾ |
| | 24 h | 7.1/92.9 (Z-Val-Val-X-OH) | cannot be determined ²⁾ |
| | 48 h | 44.1/55.9 (Z-Val-Val-X-OH) | cannot be determined ²⁾ |
| Glu(OBu*^{t}*) (mix C) | 2 h | n.d. | 27.3/72.7 |
| | 6 h | n.d. | 41.8/58.2 |
| | 24 h | n.d. | 83.4/16.6 |
| | 48 h | n.d. | 100/0 |
| Trp(Boc) (mix C) | 2h | 32.1/67.9 (H-X-Leu-OH) | 3/97 |
| | 6h | 20.1/79.9 (H-X-Leu-OH) | 10.2/89.8 |
| | 24 h | 4.9/95.1 (H-X-Leu-OH) | 69.9/30.1 |
| | 48 h | 3.5/96.5 (H-X-Leu-OH) | 100/0 |
| Val (mix D) | 2 h | n.d. | 98.8/1.2 |
| | 6 h | n.d. | 100/0 ¹⁾ |
| | 24 h | n.d. | 100/0 ¹⁾ |
| | 48 h | 0.5/99.5 (Z-Val-Val-X-OH) | 100/0 |
| Gly (mix D) | 2 h | n.d. | cannot be determined ³⁾ |
| | 6 h | n.d. | cannot be determined |
| | 24 h | n.d. | cannot be determined ³⁾ |
| | 48 h | n.d. | cannot be determined ³⁾ |
| Trp (mix D) | 2 h | 0.3/99.7 (H-X-Leu-OH) | 18.8/81.2 |
| | 6 h | 0.4/99.6 (H-X-Leu-OH) | 52.7/47.3 |
| | 24 h | 2/98 (H-X-Leu-OH) | 96.4/3.6 |
| | 48 h | 6/94 (H-X-Leu-OH) | 99.9/0.1 |
| Tyr(Bu*^{t}*) (mix D) | 2 h | 5.9/94.1 (H-X-Leu-OH) | 6.2/93.8 |
| | 6 h | 2.6/97.4 (H-X-Leu-OH) | 30.6/69.4 |
| | 24 h | 0.6/99.4 (H-X-Leu-OH) | 95.3/4.7 |
| | 48 h | 0.3/99.7 (H-X-Leu-OH) | 99.2/0.8 |

| | | | |
|---|---|---|---|
| ¹⁾ traces of substrate detected in LC/MS; ²⁾ Retention Time of substrate equal to that of Z-Val-Val-Arg(Pbf)-Leu-OH; ³⁾ Retention Time of substrate equal to that of Z-Val-Val-Tyr(Bu*^{t}*)-Leu-OH. n.d. = no endopeptidase impurity detected | | | |

### Example 6

### Scaling up of the procedure

To a stirred solution of 606 mg (1 mmol) Z-Val-Trp-Leu-OBu*^{t}* in 25 ml DMF 25 mg of Alcalase CLEA were added along with 25 ml of phosphate buffer 0.1 M pH 7. The mixture was stirred at 40°C. The reaction progress was monitored by HPLC. Extra portions of enzyme (25 mg of Alcalase CLEA each) were added after 24 hours and after 48 hours. After 6 days the reaction was stopped. The reaction mixture was acidified to pH 2 and DMF was evaporated. The product was extracted with ethyl acetate. The organic layer was dried using sodium sulphate, concentrated *in vacuo* and the final product precipitated from heptane. No endopeptidase-related impurities were identified during the analysis of the final compound both by HPLC and LC/MS.
Yield: 96.2%
HPLC purity: 96.5 a/a % (the final compound also contained 2.5 a/a % of the starting material).

## Claims

1. A process for the selective enzymatic hydrolysis of C-terminal *tert*-butyl esters of peptide substrates in the synthesis of peptides, comprising hydrolysing one or more peptide substrates comprising C-terminal *tert*-butyl esters using the protease subtilisin in any suitable form.

2. The process of claim 1, wherein the peptide substrate comprising the C-terminal *tert*-butyl ester comprises a C-terminal acyl residue which is an α-amino acyl residue from natural or synthetic origin.

3. The process of claim 2, wherein the α-amino acyl residue is selected from Ala, protected Cys, protected Asp, protected Glu, Phe, Gly, His, (protected) Lys, Leu, Met, Asn, Gln, (protected) Arg, (protected) Ser, Thr, Val, (protected) Trp and (protected) Tyr.

4. The process of any one of claims 1 to 3, wherein the peptide substrate is a di-, tri- or tetrapeptide.

5. The process of any one of claims 1 to 4, wherein the peptide substrate is prepared by solution-phase synthesis.

6. The process of claim 5, wherein the peptide substrate is prepared according to a process for rapid solution synthesis of a peptide in an organic solvent or a mixture of organic solvents, the process comprising repetitive cycles of steps (a)-(d):
a) a coupling step, using an excess of an activated carboxylic component to acylate an amino component,
b) a quenching step in which a scavenger is used to remove residual activated carboxylic functions, wherein the scavenger may also be used for deprotection of the growing peptide,
c) one or more aqueous extractions and
optionally, (d) a separate deprotection step, followed by one or more aqueous extractions,
whereby in at least one cycle in process step b an amine comprising a free anion or a latent anion is used as a scavenger of residual activated carboxylic functions.

7. The process of any one of claims 1 to 6, wherein the protease subtilisin is of the family EC 3.4.21.62.

8. The process of any one of claims 1 to 7 wherein the protease subtilisin is free subtilisin.

9. The process of any one of claims 1 to 7 wherein the protease subtilisin is cross-linked enzyme aggregate (CLEA) subtilisin.

10. The process of any one of claims 1 to 9, wherein the hydrolysis is performed in a mixture of an aqueous buffer and an organic solvent.

11. The process of claim 10, wherein the organic solvent is a polar solvent.

12. The process of claim 11, wherein the organic solvent is selected from *N,N-*dimethylformamide (DMF), *N*-methyl-2-pyrrolidone (NMP), dioxane, *N,N-*dimethylacetamide (DMA), dichloromethane (DCM), tetrahydrofuran (THF), acetonitrile, and *tert*-butanol.

13. The process of claim 12, wherein the organic solvent is DMF.

14. The process of any one of claims 10 to 13, wherein the percentage of the organic solvent in the mixture is 50-60%.

15. The process of any one of claims 1 to 14, wherein pH at which the reaction is performed is selected from the range of 6.5-10.

16. The process of claim 15, wherein the pH is selected from the range of 6.5-8.

17. The process of claim 16, wherein the pH is 7.

18. The process of any one of claims 1-17, wherein the reaction temperature for the hydrolysis is 20-60°C.

19. The process of claim 18, wherein the reaction temperature for the hydrolysis is 40°C.

20. The process of any one of claims 1-19, wherein the amount of enzyme ranges from 1 to 50 wt.% related to the peptide substrate.

21. The process of any one of claims 1-20, wherein the hydrolysis is performed by stepwise adding portions of the protease subtilisin into the reaction mixture comprising one or more peptide substrates comprising C-terminal *tert*-butyl esters.

22. A process for the convergent synthesis of a peptide from two or more peptide fragments wherein at least one of the peptide fragments is prepared according to a process of any one of claims 1 to 21.

23. A process for the stepwise enzymatic synthesis of a peptide in C-terminal direction according to a process of any one of claims 1 to 21.

24. A process for peptide synthesis comprising peptide cyclization involving the C-terminal of the linear precursor according to a process of any one of claims 1 to 21.

## Patentansprüche

1. Verfahren für die selektive enzymatische Hydrolyse der C-terminalen *tert*-Butylester von Peptidsubstraten bei der Synthese von Peptiden, umfassend die Hydrolyse von einem oder mehreren Peptidsubstraten umfassend C-terminale *tert*-Butylester unter Einsatz von Proteasesubtilisin in jeglicher geeigneter Form.

2. Verfahren gemäss Anspruch 1, wobei das Peptidsubstrat umfassend den C-terminalen *tert-*Butylester ein C-terminales Acylresiduum umfasst, welches ein α-Amino-Acylresiduum von natürlichem oder synthetischem Ursprung ist.

3. Verfahren gemäss Anspruch 2, wobei das α-Amino-Acylresiduum ausgewählt ist aus Ala, geschütztem Cys, geschütztem Asp, geschütztem Glu, Phe, Gly, His, (geschütztem) Lys, Leu, Met, Asn, Gln, (geschütztem) Arg, (geschütztem) Ser, Thr, Val, (geschütztem) Trp und (geschütztem) Tyr.

4. Verfahren gemäss irgendeinem der Ansprüche 1 bis 3, wobei das Peptidsubstrat ein Di-, Tri- oder Tetrapeptid ist.

5. Verfahren gemäss irgendeinem der Ansprüche 1 bis 4, wobei das Peptidsubstrat durch Flüssigphasen-Synthese hergestellt wird.

6. Verfahren gemäss Anspruch 5, wobei das Peptidsubstrat gemäss einem Verfahren zur schnellen Flüssigphasensynthese eines Peptids in einem organischen Lösungsmittel oder einer Mischung von organischen Lösungsmitteln hergestellt wird, wobei das Verfahren sich wiederholende Zyklen der Schritte (a)-(d) umfasst:
a) einen Kopplungsschritt, einsetzend einen Überschuss einer aktivierten Carboxyl-Komponente, um eine Aminokomponente zu acylieren,
b) einen Quenchschritt, bei dem ein Radikalfänger eingesetzt wird, um residuell aktivierte Carboxylfunktionen zu entfernen, wobei der Radikalfänger auch zum Entschützen des wachsenden Peptids eingesetzt werden kann,
c) eine oder mehrere wässrige Extraktionen und
optional (d) einen getrennten Entschützungsschritt, gefolgt von einer oder mehreren wässrigen Extraktionen, wobei bei mindestens einem Zyklus im Verfahrensschritt b ein Amin, umfassend ein freies Anion oder ein latentes Anion, als ein Radikalfänger von residuell aktivierten Carboxylfunktionen eingesetzt wird.

7. Verfahren gemäss irgendeinem der Ansprüche 1 bis 6, wobei das Proteasesubtilisin aus der Familie EC 3.4.21.62 ist.

8. Verfahren gemäss irgendeinem der Ansprüche 1 bis 7, wobei das Proteasesubtilisin freies Subtilisin ist.

9. Verfahren gemäss irgendeinem der Ansprüche 1 bis 7, wobei das Proteasesubtilisin kreuzvernetztes Enzyme-Aggregat (CLEA) Subtilisin ist.

10. Verfahren gemäss irgendeinem der Ansprüche 1 bis 9, wobei die Hydrolyse in einer Mischung aus einem wässrigen Puffer und einem organischen Lösungsmittel durchgeführt ist.

11. Verfahren gemäss Anspruch 10, wobei das organische Lösungsmittel ein polares Lösungsmittel ist.

12. Verfahren gemäss Anspruch 11, wobei das organische Lösungsmittel ausgewählt ist aus *N,N-*Dimethylformamid (DMF), *N*-Methyl-2-pyrrolidon (NMP), Dioxan, *N,N*-Dimethylacetamid (DMA), Dichlormethan (DCM), Tetrahydrofuran (THF), Acetonitril, und *tert*-Butanol.

13. Verfahren gemäss Anspruch 12, wobei das organische Lösungsmittel DMF ist.

14. Verfahren gemäss irgendeinem der Ansprüche 10 bis 13, wobei der Prozentanteil des organischen Lösungsmittels in der Mischung zwischen 50-60% liegt.

15. Verfahren gemäss irgendeinem der Ansprüche 1 bis 14, wobei der pH-Wert, an der die Reaktion durchgeführt wird, ausgewählt wird aus dem Bereich zwischen 6.5 und 10.

16. Verfahren gemäss Anspruch 15, wobei der pH-Wert ausgewählt wird aus dem Bereich zwischen 6.5 und 8.

17. Verfahren gemäss Anspruch 16, wobei der pH-Wert 7 ist.

18. Verfahren gemäss irgendeinem der Ansprüche 1 bis 17, wobei die Reaktionstemperatur für die Hydrolyse zwischen 20 und 60 Grad Celsius ist.

19. Verfahren gemäss Anspruch 18, wobei die Reaktionstemperatur für die Hydrolyse 40 Grad Celsius ist.

20. Verfahren gemäss irgendeinem der Ansprüche 1 bis 19, wobei die Menge des Enzyms zwischen 1 und 50 Gewichtsprozent in Bezug auf das Peptidsubstrat liegt.

21. Verfahren gemäss irgendeinem der Ansprüche 1 bis 20, wobei die Hydrolyse durchgeführt wird durch schrittweises Hinzufügen von Portionen des Proteasesubtilisins in die Reaktionsmischung, umfassend eines oder mehrere Peptidsubstrate umfassend C-terminale *tert-*Butylester.

22. Verfahren für die konvergente Synthese eines Peptids aus zwei oder mehreren Peptidfragmenten, wobei mindestens eines der Peptidfragmente gemäss einem Verfahren gemäss irgendeinem der Ansprüche 1 bis 21 hergestellt wird.

23. Verfahren für die schrittweise enzymatische Synthese eines Peptids in C-terminaler Richtung gemäss einem Verfahren gemäss irgendeinem der Ansprüche 1 bis 21.

24. Verfahren der Peptidsynthese, umfassend Peptidzyklisierung, einbindend das C-Terminale des linearen Precursors gemäss einem Verfahren gemäss irgendeinem der Ansprüche 1 bis 21.

## Revendications

1. Un procédé pour l'hydrolyse enzymatique sélective d'esters *tert*-butyle C-terminaux de substrats peptidiques lors de la synthèse de peptides, comprenant l'hydrolyse de un ou plusieurs substrats peptidiques comprenant des esters *tert-*butyle C-terminaux, faisant emploi de protéase subtilisine sous une quelconque forme convenable.

2. Le procédé selon la revendication 1, dans lequel le substrat peptidique comprenant l'ester tert-butyle C-terminaux comprend un résidu acyl C-terminal qui consiste en un résidu α-amino acyl ayant une origine naturelle ou synthétique.

3. Le procédé selon la revendication 2, dans lequel le résidu α-amino acyl est sélectionné parmi Ala, Cys protégé, Asp protégé, Glu protégé, Phe, Gly, His, Lys (protégé), Leu, Met, Asn, Gln, Arg (protégé), Ser (protégé), Thr, Val, Trp (protégé) et Tyr (protégé).

4. Le procédé selon l'une quelconque des revendications 1 à 3, dans lequel le substrat peptidique est un di-, tri- ou tetrapeptide.

5. Le procédé selon l'une quelconque des revendications 1 à 4, dans lequel le substrat peptidique est préparé par synthèse en phase liquide.

6. Le procédé selon la revendication 5, dans lequel le substrat peptidique est préparé selon un procédé de synthèse en solution rapide d'un peptide dans un solvant organique ou un mélange de solvants organiques, le procédé comprenant des cycles répétitifs des étapes (a) à (d):
(a) une étape de couplage, faisant emploi d'un excès d'un dérivé carboxylique activé pour acyler un dérivé amino,
(b) une étape d'atténuation ("quenching") dans laquelle est employé un scavenger pour éliminer les fonctions carboxyliques activées résiduelles, ce scavenger pouvant également être utilisé pour la déprotection des peptide en croissance,
(c) une ou plusieurs extractions aqueuses et éventuellement,
(d) une étape de déprotection distincte, suivie par une ou plusieurs extractions aqueuses, où dans au moins un des cycles de l'étape (b) du procédé est employée une amine comprenant un anion libre ou un anion latent agissant en tant que scavenger à l'égard des fonctions carboxyliques activées résiduelles.

7. Le procédé selon l'une quelconque des revendications 1 à 6, dans lequel la protéase subtilisine appartient à la famille EC 3.4.21.62.

8. Le procédé selon l'une quelconque des revendications 1 à 7, dans lequel la protéase subtilisine est de la subtilisine libre.

9. Le procédé selon l'une quelconque des revendications 1 à 7 dans lequel la protéase subtilisine est un agrégat d'enzyme subtilisine réticulée ("cross-linked enzyme aggregate (CLEA)").

10. Le procédé selon l'une quelconque des revendications 1 à 9, dans lequel l'hydrolyse est mise en oeuvre dans un mélange de tampon aqueux et de solvant organique.

11. Le procédé selon la revendication 10, dans lequel le solvant organique est un solvant polaire.

12. Le procédé selon la revendication 11, dans lequel le solvant organique est sélectionné dans le groupe comprenant *N,N* -diméthylformamide (DMF), *N-*méthyl-2-pyrrolidone (NMP), dioxane, *N,N*-diméthylacétamide (DMA), dichlorométhane (DCM), tetrahydrofuran (THF), acétonitrile et *tert*-butanol.

13. Le procédé selon la revendication 12, dans lequel le solvant organique consiste en le DMF.

14. Le procédé selon l'une quelconque des revendications 10 à 13, dans lequel le pourcentage de solvant organique dans le mélange est de 50 à 60%.

15. Le procédé selon l'une quelconque des revendications 1 à 14, dans lequel le pH auquel la réaction est mise en oeuvre est sélectionné dans la plage de 6,5 à 10.

16. Le procédé selon la revendication 15, dans lequel le pH est sélectionné dans la plage de 6,5 à 8.

17. Le procédé selon la revendication 16, dans lequel le pH est de 7.

18. Le procédé selon l'une quelconque des revendications 1 à 17, dans lequel la température de réaction lors de l'hydrolyse est de 20 à 60°C.

19. Le procédé selon la revendication 18, dans lequel la température de réaction lors de l'hydrolyse est de 40°C.

20. Le procédé selon l'une quelconque des revendications 1-19, dans lequel la proportion d'enzyme est comprise entre 1 et 50% en poids exprimé par rapport au substrat peptidique.

21. Le procédé selon l'une quelconque des revendications 1 à 20, dans lequel l'hydrolyse est mise en oeuvre par addition par étapes de portions de protéase subtilisine dans le mélange réactionnel comprenant un ou plusieurs substrats peptidiques comprenant des esters *tert*-butyle C-terminaux.

22. Un procédé pour la synthèse convergente d'un peptide à partir de deux ou plusieurs fragments peptidiques dans lequel au moins un des fragments peptidiques est préparé par un procédé selon l'une quelconque des revendications 1 à 21.

23. Un procédé pour la synthèse enzymatique par étapes d'un peptide en direction du C- terminal, par un procédé selon l'une quelconque des revendications 1 à 21.

24. Un procédé pour la synthèse de peptide comprenant la cyclisation de peptide impliquant le C-terminal du précurseur linéaire d'après un procédé selon l'une quelconque des revendications 1 à 21.
